# FASCICULE DE BREVET EUROPEEN

(11) **EP 0 628 305 B1**
(45) Date de publication et mention de la délivrance du brevet: **07.05.1997**
(21) Numéro de dépôt: 94401295.4
(22) Date de dépôt: 09.06.1994
(51) Int. Cl.: A61K 7/50

(54) **Composition cosmétique ou dermatologique sous forme d'une émulsion huile-dans-l'eau stable contenant au moins une huile végétale constituée d'au moins 40 % de triglycérides d'acide linoléique**
Kosmetisches oder dermatologisches Präparat in Form einer stabilen Wasser-in-Öl-Emulsion welche mindestens ein pflanzliches Öl enthält, das mindestens aus 40% Linolsäure Triglycerid besteht
Stable oil-in-water emulsion type cosmetic or dermatologic composition containing at least one vegetable oil comprising at least 40% triglycerides derived from linolic acid

(30) Priorité: 10.06.1993 FR 9307003
(43) Date de publication de la demande: 14.12.1994
(73) Titulaire: L'OREAL, 75008 Paris (FR)
(72) Inventeur: Khayat, Carine, F-94210 La Varenne (FR); Candau, Didier, F-91570 Bievres (FR)
(74) Mandataire: Stalla-Bourdillon, Bernard

(56) Documents cités:
- WO-A-92/06778

## Description

La présente invention a pour objet une composition sous forme d'une émulsion huile-dans-eau (H/E) stable essentiellement constituée d'au moins une huile végétale à haute teneur en acide linoléique, d'un tensioactif à base d'alcools gras et d'un agent gélifiant, et son utilisation dans différentes applications cosmétiques et dermatologiques.

Les huiles végétales, telles que l'huile d'amande douce ou l'huile de noisette, sont connues pour leurs propriétés cosmétiques mais leurs utilisations pour la préparation d'émulsions présentent toutefois de sérieuses difficultés, notamment en ce qui concerne leur stabilité. Pour résoudre ce problème et pour éviter l'emploi de tensioactifs plus ou moins irritants, il a été proposé dans la demande WO 92/06778, l'utilisation d'une composition auto-émulsionnable à base d'alcools gras et d'alkylpolyosides.

Toutefois, les huiles décrites dans ce brevet sont des huiles à faible pourcentage en triglycérides d'acide linoléique. Or, cet acide, en tant qu'acide gras insaturé, joue un rôle très important dans le maintien des structures lipidiques des espaces intercellulaires du stratum corneum ainsi que dans la restauration de la fonction de barrière des peaux sèches. Dans la mesure où l'acide linoléique n'est pas synthétisé par le corps humain, celui-ci doit donc être apporté au tissu cutané par voie topique ou éventuellement par voie orale sous forme d'acide libre ou sous forme de triglycérides.

Il était donc souhaitable de pouvoir obtenir des émulsions stables à partir d'huiles végétales particulièrement riches en acide linoléique.

L'utilisation de la composition auto-émulsionnable, selon WO 92/06778, ne permet pas cependant d'obtenir des émulsions suffisamment stables dans le temps avec ce type d'huiles.

Il a maintenant été constaté de façon surprenante et inattendue qu'en associant ces huiles végétales à la composition auto-émulsionnable selon le brevet WO 92/06778, en présence d'un co-émulsionnant et d'un agent gélifiant, il était possible d'obtenir des émulsions fines et stables, c'est-à-dire ne présentant pas de déphasage après deux mois à 45°C.

La bonne stabilité des émulsions (H/E) selon l'invention a pu être obtenue notamment grâce à la présence de l'agent gélifiant utilisé en une proportion bien déterminée et choisi parmi des agents gélifiants naturels.

La présente invention a donc pour objet une composition cosmétique ou dermatologique sous forme d'une émulsion huile-dans-eau stable, caractérisée par le fait qu'elle contient :
(a) de 15 à 50 % en poids d'au moins une huile végétale constituée d'au moins 40 % de triglycérides d'acide linoléique,
(b) de 2 à 7 % en poids d'une composition ou association auto-émulsionnable comprenant de 60 à 90 % en poids d'au moins un alcool gras ayant de 12 à 22 atomes de carbone de 10 à 40 % en poids d'au moins un alkylpolyoside dont la chaîne alkyle a de 12 à 22 atomes de carbone et de 0 à 5 % en poids de polyoside,
(c) de 0,5 à 5 % en poids d'un co-émulsionnant choisi parmi au moins un alcool gras saturé ayant de 16 à 32 atomes de carbone, un acide gras saturé ayant de 16 à 32 atomes de carbone et leurs mélanges,
(d) de 0,1 à 1 % en poids d'un agent gélifiant, et
le reste étant essentiellement constitué par une phase aqueuse.

Selon un mode de réalisation préféré, le rapport pondéral entre l'huile végétale et l'alkylpolyoside de l'association auto-émulsionnable est supérieur ou égal à 10.

Parmi les huiles végétales comprenant au moins 40 % de triglycérides d'acide linoléique utilisables selon l'invention, on peut citer l'huile de germes de blé, l'huile de germes de maïs, l'huile de soja, l'huile de tournesol, l'huile de coton, l'huile de luzerne, l'huile de pavot, l'huile de potimarron, l'huile de sésame, l'huile de pépins de raisin, l'huile d'onagre, l'huile de millet, l'huile d'orge, l'huile de quinoa, l'huile de seigle, l'huile de carthame, l'huile de bancoulier et l'huile de passiflore.

Les pourcentages en triglycérides des acides palmitique, oléique et linoléique constituant ces huiles végétales sont respectivement compris entre environ 0 et 10 %, 20 et 30%, et 40 et 70 %.

Selon un mode de réalisation préféré de l'invention, la proportion en huiles végétales est comprise entre 15 et 40 % en poids par rapport au poids total de l'émulsion.

Parmi les associations ou compositions auto-émulsionnables décrites dans WO 92/06778, on peut notamment citer le produit commercialisé sous la dénomination de "Montanov 68" par la Société Seppic qui est essentiellement constitué par l'association d'environ 77 % d'alcool cétylstéarylique (C₁₆-C₁₈) et d'environ 23 % de cétéarylglucosides.

Selon un mode de réalisation particulier de l'invention, le radical alkyle de l'alkylpolyoside est identique à celui de l'alcool gras de la composition auto-émulsionnable.

Selon un mode de réalisation préféré de l'invention, la proportion en composition auto-émulsionnable est comprise entre 2 et 6 % en poids par rapport au poids total de l'émulsion.

Parmi les alcools gras ayant de 16 à 32 atomes de carbone utilisés comme co-émulsionnants, on peut citer l'alcool cétylique, l'alcool stéarylique et leurs mélanges ainsi qu'un mélange d'octacosanol et d'alcools gras ayant de 24 à 32 atomes de carbone.

Parmi les acides gras ayant de 16 à 32 atomes de carbone utilisés comme co-émulsionnants, on peut citer l'acide palmitique et l'acide stéarique.

Comme co-émulsionnant appartenant au groupe des acides gras ayant de 16 à 32 atomes de carbone, on peut utiliser selon l'invention un produit d'origine naturelle à savoir la cire de sumac qui contient environ 40 % d'acides gras en C₁₆ et 50 % d'acides gras en C₁₈.

Selon une forme de réalisation préférée, la proportion en co-émulsionnant est comprise entre 0,5 et 3 % en poids par rapport au poids total de l'émulsion.

Parmi les agents gélifiants utilisables selon l'invention, on peut citer les farines de graines telles que la gomme de guar et la gomme de caroube, les exsudats d'arbres tels que la gomme arabique, le karaya, l'adragante et le ghatty, les extraits d'algues marines tels que les carraghénates, les alginates et l'agar-agar, les exsudats de micro-organismes tels que la gomme de xanthane, les dérivés de cellulose et les mélanges de ceux-ci. Parmi ces agents gélifiants, ceux particulièrement préférés sont les gommes de xanthane et les alginates, notamment l'alginate de propylèneglycol.

Les émulsions selon l'invention peuvent contenir en outre, en vue d'augmenter leurs qualités cosmétiques, une cire cosmétique telle que par exemple de la cire de riz, de la cire de candellila, de la cire du Japon, ces cires ayant un point de fusion compris entre 46 et 52°C. La proportion de cire est généralement comprise entre 0,5 et 3 %, et de préférence entre 1 et 2 % en poids par rapport au poids total de l'émulsion.

Les émulsions selon l'invention peuvent également contenir au moins un adjuvant conventionnel. Parmi ceux-ci, on peut citer notamment les filtres UV, les vitamines, les huiles essentielles, les protéines végétales, les antioxydants, les conservateurs, les parfums, les céramides, les agents hydratants, les agents lubrifiants, les polysaccharides, les charges et divers principes actifs.

Les compositions selon l'invention sous forme d'émulsions (H/E) peuvent être utilisées dans différentes applications cosmétiques ou dermatologiques par exemple sous forme de crèmes pour le visage ou pour le corps, de laits démaquillants, de laits pour le corps ou de masques pour le visage. Ces compositions peuvent être également utilisées pour le maquillage après addition de pigments, notamment sous forme de fonds de teint. Elles peuvent être utilisées également comme crèmes solaires après addition de filtres UV-B et/ou UV-A.

On va maintenant donner à titre d'illustration plusieurs exemples de compositions selon l'invention sous forme d'émulsions huile-dans-eau.

### EXEMPLES DE COMPOSITIONS

### EXEMPLE 1 : Crème hydratante

| *Phase grasse A :* Mélange (A₁): | |
|---|---|
| - Composition auto-émulsionnable commercialisée sous la dénomination de "Montanov 68" par la Société Seppic | 2,5 g |
| - Alcool cétylique | 1 g |
| - Cire de sumac | 1,5 g |

| Mélange (A₂): | |
|---|---|
| - Huile de germes de maïs | 24 g |
| - Antioxydant q.s. | |

| *Phase aqueuse B:* | |
|---|---|
| - Gomme de xanthane | 0,2 g |
| - Conservateurs q.s. | |
| - Eau | 25 g |

On prépare la *Phase grasse A* de la manière suivante :

On chauffe séparément le Mélange (A₁) à 80°C, et le Mélange (A₂) à 70°C sous agitation magnétique, puis on verse le Mélange (A₁) dans le Mélange (A₂) et on homogénéise.

On prépare la *Phase aqueuse B* de la manière suivante :

On chauffe l'eau à 80°C et y solubilise les conservateurs, puis on introduit sous agitation magnétique l'agent gélifiant jusqu'à obtention d'un gel opaque homogène. On refroidit alors le mélange à 70°C.

On verse ensuite lentement la *Phase A* dans la *Phase B*, sous agitation, à 70°C, pendant environ 5 minutes, puis on laisse refroidir le mélange à 30°C. On ajoute alors au mélange obtenu précédemment de l'eau en quantité suffisante pour 100 g et agite jusqu'à obtention d'une crème homogène.

La crème obtenue est très souple, lisse, d'un toucher agréable et laisse la peau mate après application. Sa viscosité est comprise entre 6 et 10 x 10⁻¹ Pa.s (6 et 10 poises).

Elle présente une excellente stabilité en centrifugation à 3000 trs/mn pendant une heure, et après conservation pendant 2 mois à 45°C.

### EXEMPLE 2 : Lait hydratant

| *Phase grasse A :* Mélange (A₁): | |
|---|---|
| - Composition auto-émulsionnable commercialisée sous la dénomination de "Montanov 68" par la Société Seppic | 2,3 g |
| - Alcool stéarylique | 1 g |

| Mélange (A₂): | |
|---|---|
| - Huile de soja | 16,7 g |
| - Antioxydant q.s. | |

| *Phase aqueuse B :* | |
|---|---|
| - Gomme de xanthane | 0,6 g |
| - Conservateurs q.s. | |
| - Eau | 25 g |

Ce lait hydratant est obtenu selon le même mode opératoire que celui décrit à l'exemple 1 par mélange des *Phases A* et *B* ci-dessus et addition d'une quantité suffisante d'eau pour 100 g.

Il est très facile à appliquer sur le visage et sur le corps et est, de plus, très doux à l'application.

Sa viscosité est comprise entre 210 et 290 cps et présente les mêmes critères de stabilité que ceux décrits pour la crème de l'exemple 1.

### EXEMPLE 3: Crème nettoyante

| *Phase grasse A :* Mélange (A₁) : | |
|---|---|
| - Composition auto-émulsionnable commercialisée sous la dénomination de "Montanov 68" par la Société Seppic | 4,25 g |
| - Alcool cétylique | 0,75 g |
| - Cire du Japon | 0,5 g |

| Mélange (A₂): | |
|---|---|
| - Huile de germes de maïs | 20 g |
| - Huile de germes de blé | 5 g |

| *Phase aqueuse B :* | |
|---|---|
| - Alginate de propylène glycol | 0,6 g |
| - Conservateurs q.s. | |
| - Eau | 25 g |

On procède à la préparation de cette crème selon le même mode opératoire que celui décrit à l'exemple 1. Lorsque la température est redescendue à 30°C, on ajoute sous agitation, au mélange, une quantité d'eau suffisante pour 95 g, puis 5 g de kaolin et homogénéise.

La crème nettoyante obtenue est blanche, présente un bon pouvoir nettoyant et est très douce, à l'application et après l'application. Sa viscosité est comprise entre 15 et 25 poises. Cette crème présente une excellente stabilité en centrifugation à 3.000 trs/mn pendant une heure et après conservation pendant 2 mois à toute température.

### EXEMPLE 4 : Lait nettoyant

| *Phase grasse A :* Mélange (A₁): | |
|---|---|
| - Composition auto-émulsionnable commercialisée sous la dénomination de "Montanov 68" par la Société Seppic | 2,45g |
| - Acide stéarique | 1g |
| - Acide palmitique | 0,8 g |

| Mélange (A₂): | |
|---|---|
| - Huile de germes de maïs | 24 g |
| - Antioxydants q.s. | |

| *Phase aqueuse B :* | |
|---|---|
| - Gomme de xanthane | 0,2 g |
| - Protéine de blé | 0,5 g |
| - Conservateurs q.s. | |
| - Eau | 25 g |

Ce lait nettoyant est obtenu selon le même mode opératoire que celui décrit à l'exemple 1 par mélange des *Phases A* et *B* ci-dessus et addition d'une quantité suffisante d'eau pour 100 g.

Il est très doux à l'application.

Sa viscosité est comprise entre 220 et 290 x 10⁻³ Pa.s (220 et 290 cP) et présente les mêmes critères de stabilité que ceux décrits pour la crème de l'exemple 1.

## Revendications

1. Composition cosmétique ou dermatologique sous forme d'une émulsion huile-dans-eau stable, caractérisée par le fait qu'elle contient :
(a) de 15 à 50 % en poids d'au moins une huile végétale constituée d'au moins 40 % de triglycérides d'acide linoléique,
(b) de 2 à 7 % en poids d'une composition auto-émulsionnable comprenant de 60 à 90 % en poids d'au moins un alcool gras ayant de 12 à 22 atomes de carbone, de 10 à 40 % en poids d'au moins un alkylpolyoside dont la chaîne alkyle a de 12 à 22 atomes de carbone et de 0 à 5 % en poids de polyoside,
(c) de 0,5 à 5 % en poids d'un co-émulsionnant choisi parmi au moins un alcool gras saturé ayant de 16 à 32 atomes de carbone, un acide gras saturé ayant de 16 à 32 atomes de carbone et leurs mélanges,
(d) de 0,1 à 1 % en poids d'un agent gélifiant, et
le reste étant essentiellement constitué par une phase aqueuse.

2. Composition selon la revendication 1, caractérisée par le fait que le rapport pondéral entre l'huile végétale et l'alkylpolyoside de la composition auto-émulsionnable est supérieur ou égal à 10.

3. Composition selon la revendication 1, caractérisée par le fait que l'alcool gras ayant de 12 à 22 atomes de carbone de la composition auto-émulsionnable est l'alcool cétylstéarylique (C₁₆-C₁₈) et l'alkylpolyoside est du cétéarylglucoside.

4. Composition selon la revendication 1, caractérisée par le fait que l'huile végétale est choisie dans le groupe constitué par: l'huile de germes de blé, l'huile de germes de maïs, l'huile de soja, l'huile de tournesol, l'huile de coton, l'huile de luzerne, l'huile de pavot, l'huile de potimarron, l'huile de sésame, l'huile de pépins de raisin, l'huile d'onagre, l'huile de millet, l'huile d'orge, l'huile de quinoa, l'huile de seigle, l'huile de carthame, l'huile de bancoulier et l'huile de passiflore.

5. Composition selon l'une quelconque des revendications précédentes, caractérisée par le fait que l'huile végétale est présente en une proportion comprise entre 15 et 40 % en poids par rapport au poids total de l'émulsion.

6. Composition selon l'une quelconque des revendications précédentes, caractérisée par le fait que la composition auto-émulsionnable est présente en une proportion comprise entre 2 et 6 % en poids par rapport au poids total de l'émulsion.

7. Composition selon l'une quelconque des revendications précédentes, caractérisée par le fait que le co-émulsionnant est choisi dans le groupe constitué par : l'alcool cétylique, l'alcool stéarylique et leurs mélanges, un mélange d'octacosanol et d'alcools gras ayant de 24 à 32 atomes de carbone ; l'acide palmitique et l'acide stéarique.

8. Composition selon l'une quelconque des revendications précédentes, caractérisée par le fait que le co-émulsionnant est présent en une proportion comprise entre 0,5 et 3 % en poids par rapport au poids total de l'émulsion.

9. Composition selon l'une quelconque des revendications précédentes, caractérisée par le fait que l'agent gélifiant est choisi dans le groupe constitué par: les farines de graines, les exsudats d'arbres, les extraits d'algues marines, les exsudats de micro-organismes et les dérivés de cellulose.

10. Composition selon l'une quelconque des revendications précédentes, caractérisée par le fait qu'elle contient en outre, de 0,5 à 3 % en poids par rapport au poids total de l'émulsion d'au moins une cire cosmétique choisie dans le groupe constitué par: la cire de riz, la cire de candellila et la cire du Japon.

11. Composition selon l'une quelconque des revendications précédentes, caractérisée par le fait qu'elle contient en outre au moins un adjuvant choisi parmi les filtres, les vitamines, les huiles essentielles, les protéines végétales, les antioxydants, les conservateurs, les parfums, les céramides, les agents hydratants, les agents lubrifiants, les polysaccharides, les charges et divers principes actifs.

## Claims

1. Cosmetic or dermatological composition in the form of a stable oil-in-water emulsion, characterized in that it contains:
(a) from 15 to 50% by weight of at least one vegetable oil consisting of at least 40% linoleic acid triglycerides,
(b) from 2 to 7% by weight of a self-emulsifiable composition comprising from 60 to 90% by weight of at least one fatty alcohol having from 12 to 22 carbon atoms, from 10 to 40% by weight of at least one alkylpolysaccharide whose alkyl chain has from 12 to 22 carbon atoms, and from 0 to 5% by weight of polysaccharide,
(c) from 0.5 to 5% by weight of a coemulsifying agent chosen from at least one saturated fatty alcohol having from 16 to 32 carbon atoms, a saturated fatty acid having from 16 to 32 carbon atoms and their mixtures,
(d) from 0.1 to 1% by weight of a gelling agent, and
the remainder consisting essentially of an aqueous phase.

2. Composition according to Claim 1, characterized in that the ratio by weight of the vegetable oil to the alkylpolysaccharide of the self -emulsifiable composition is greater than or equal to 10.

3. Composition according to Claim 1, characterized in that the fatty alcohol having from 12 to 22 carbon atoms of the self-emulsifiable composition is cetyl/stearyl alcohol (C₁₆-C₁₈) and the alkylpolysaccharide is cetearylglucoside.

4. Composition according to Claim 1, characterized in that the vegetable oil is chosen from the group consisting of: wheat germ oil, maize germ oil, soybean oil, sunflower oil, cottonseed oil, lucerne oil, poppy oil, pumpkinseed oil, sesame oil, rapeseed oil, evening primrose oil, millet oil, barley oil, quinoa oil, rye oil, safflower oil, candlenut oil and passionflower oil.

5. Composition according to any one of the preceding claims, characterized in that the vegetable oil is present in a proportion of between 15 and 40% by weight with respect to the total weight of the emulsion.

6. Composition according to any one of the preceding claims, characterized in that the self-emulsifiable composition is present in a proportion of between 2 and 6% by weight with respect to the total weight of the emulsion.

7. Composition according to any one of the preceding claims, characterized in that the coemulsifying agent is chosen from the group consisting of: cetyl alcohol, stearyl alcohol and their mixtures, a mixture of octacosanol and fatty alcohols having from 24 to 32 carbon atoms, palmitic acid and stearic acid.

8. Composition according to any one of the preceding claims, characterized in that the coemulsifying agent is present in a proportion of between 0.5 and 3% by weight with respect to the total weight of the emulsion.

9. Composition according to any one of the preceding claims, characterized in that the gelling agent is chosen from the group consisting of: seed flours, tree exudates, marine algae extracts, microorganism exudates and cellulose derivatives.

10. Composition according to any one of the preceding claims, characterized in that it additionally contains from 0.5 to 3% by weight, with respect to the total weight of the emulsion, of at least one cosmetic wax chosen from the group consisting of: rice wax, candelilla wax and Japan wax.

11. Composition according to any one of the preceding claims, characterized in that it additionally contains at least one adjuvant chosen from screening agents, vitamins, essential oils, plant proteins, anti-oxidizing agents, preserving agents, fragrances, ceramides, moisturizing agents, lubricating agents, polysaccharides, fillers and various active principles.

## Patentansprüche

1. Kosmetische oder dermatologische Zubereitung in Form einer stabilen Öl-in-Wasser-Emulsion, dadurch gekennzeichnet, daß sie
(a) 15 bis 50 Gew.-% mindestens eines pflanzlichen Öls, das aus mindestens 40 % Linolsäuretriglyceriden besteht,
(b) 2 bis 7 Gew.-% einer selbstemulgierbaren Zusammensetzung, die 60 bis 90 Gew.-% mindestens eines fetten Alkohols mit 12 bis 22 Kohlenstoffatomen, 10 bis 40 Gew.-% mindestens eines Alkylpolyosids, dessen Alkylkette 12 bis 22 Kohlenstoffatome und 0 bis 5 Gew.-% Polyosid aufweist,
(c) 0,5 bis 5 Gew.-% eines Co-Emulgators, der aus mindestens einem gesättigten Fettalkohol mit 16 bis 32 Kohlenstoffatomen, einer gesättigten Fettsäure mit 16 bis 32 Kohlenstoffatomen, sowie deren Gemischen ausgewählt ist,
(d) 0,1 bis 1 Gew.-% eines Geliermittels,
enthält,
wobei der Rest im wesentlichen aus einer wäßrigen Phase besteht.

2. Zubereitung nach Anspruch 1, dadurch gekennzeichnet, daß das Gewichtsverhältnis von pflanzlichem Öl und dem Alkylpolyosid der selbstemulgierbaren Zusammensetzung mehr oder gleich 10 beträgt.

3. Zubereitung nach Anspruch 1, dadurch gekennzeichnet, daß der Fettalkohol mit 12 bis 22 Kohlenstoffatomen in der selbstemulgierbaren Zusammensetzung den C₁₆-C₁₈-Cetylstearylalkohol und das Alkylpolyosid das Cetearylglucosid darstellen.

4. Zubereitung nach Anspruch 1, dadurch gekennzeichnet, daß das pflanzliche Öl aus der aus Weizenkeimöl, Maiskeimöl, Sojaöl, Sonnenblumenöl, Baumwoll(samen)öl, Luzernenöl, Mohnöl, (Poti)Maronenöl, Sesamöl, Traubenkernöl, Onageröl, Hirseöl, Gerstenöl, Chinaöl, Roggenöl, Färberdistelöl, Bancoulieröl (aus Aleurites moluccana) sowie Passionsblumenöl bestehenden Gruppe ausgewählt ist.

5. Zubereitung nach einem der vorstehenden Ansprüche, dadurch gekennzeichnet, daß das pflanzliche Öle in einem Mengenverhältnis zwischen 15 und 40 Gew.-% in bezug auf das Gesamtgewicht der Emulsion vorhanden ist.

6. Zubereitung nach einem der vorstehenden Ansprüche, dadurch gekennzeichnet, daß die selbstemulgierbare Zusammensetzung in einem Mengenverhältnis vorhanden ist, das zwischen 2 und 6 Gew.-% in bezug auf das Gesamtgewicht der Emulsion beträgt.

7. Zubereitung nach einem der vorstehenden Ansprüche, dadurch gekennzeichnet, daß der Co-Emulgator aus der aus Cetylalkohol, Stearylalkohol sowie deren Gemischen, einer Mischung aus Octacosanol und Fettalkoholen mit 24 bis 32 Kohlenstoffatomen, sowie aus der Palmitinsäure und Stearinsäure bestehenden Gruppe ausgewählt ist.

8. Zubereitung nach einem der vorstehenden Ansprüche, dadurch gekennzeichnet, daß der Co-Emulgator in einem Mengenverhältnis vorhanden ist, das zwischen 0,5 und 3 Gew.-% in bezug auf das Gesamtgewicht der Emulsion beträgt.

9. Zubereitung nach einem der vorstehenden Ansprüche, dadurch gekennzeichnet, daß das Geliermittel aus der aus Kornmehlen, Baumauszügen, Meeresalgenextrakten, Auszügen von Mikroorganismen und Cellulosederivaten bestehenden Gruppe ausgewählt ist.

10. Zubereitung nach einem der vorstehenden Ansprüche, dadurch gekennzeichnet, daß sie zusätzlich noch 0,5 bis 3 Gew.-% mindestens eines kosmetischen Wachses in bezug auf das Gesamtgewicht der Emulsion enthält, das aus der aus Reiswachs, Candellilawachs und Japanwachs bestehenden Gruppe ausgewählt ist.

11. Zubereitung nach einem der vorstehenden Ansprüche, dadurch gekennzeichnet, daß sie zusätzlich noch mindestens einen Hilfsstoff enthält, der aus Filtersubstanzen, Vitaminen, essentiellen Ölen, pflanzlichen Proteinen, Antioxidantien, Konservierungsmitteln, Duftstoffen, Ceramiden, Hydratisierungsmitteln, Gleitmitteln, Polysacchariden, Trägerstoffen und verschiedenen Wirkstoffen ausgewählt ist.
